# EUROPÄISCHE PATENTANMELDUNG

(11) **EP 0 950 420 A2**
(43) Veröffentlichungstag der Anmeldung: **20.10.1999**
(21) Anmeldenummer: 99106161.5
(22) Anmeldetag: 07.04.1999
(51) Int. Cl.: A61L 25/00

(54) **Tricalciumphosphathaltige Biozementpasten mit Kohäsionspromotoren**

(30) Priorität: 16.04.1998 DE 19816858
(71) Anmelder: MERCK PATENT GmbH, 64293 Darmstadt (DE)
(72) Erfinder: Wenz, Robert, Dr., 61206 Wöllstadt (DE); Driessens, C.M., Prof.Dr., 6109 AH Ohe en Laak (NL)

(57) **Zusammenfassung**

Die Erfindung betrifft Biozementpasten auf Basis von Tricalciumphosphat sowie Materialien, welche geeignet sind, die Desintegrationstendenzen der aushärtbaren Pasten zu verhindern bzw. zu verringern und ihre Kohäsionseigenschaften zu verbessern, wodurch eine verbesserte Verarbeitung besagter Pasten bei der Herstellung von synthetischen Knochenzementen gewährleistet wird.

## Beschreibung

Die Erfindung betrifft Biozementpasten auf Basis Von Tricalciumphosphat sowie Materialien, welche geeignet sind, die Desintegrationstendenzen der aushärtbaren Pasten zu verhindern bzw. zu verringern und ihre Kohäsionseigenschaften zu verbessern, wodurch eine verbesserte Verarbeitung besagter Pasten bei der Herstellung von synthetischen Knochenzementen gewährleistet wird.
Die erfindungsgemäßen Kohäsionspromotoren werden dabei bevorzugt ausgesucht aus der Gruppe folgender Verbindungen bzw. Verbindungsklassen: Hydroxyethylstärke, Cyclodextrine, Alginate, Dextransulfate, Polyvinylpyrrolidon und Hyaluronsäure.

Der Vorteil der erfindungsgemäßen Pasten ist es, daß sie nicht inhomogen werden oder sich auflösen, wenn sie in Kontakt mit wäßrigen Lösungen, wie Kochsalzlösungen, und Körperflüssigkeiten, wie z. B. Blut kommen. Ferner desintegrieren solche Pasten nicht oder nur stark vermindert, wenn sie mittels Spritzen unter in der Regel erhöhtem Druck in den defekten Knochenraum injiziert werden.

Natürlich vorkommendes Knochenmaterial besteht aus Calciumphosphat von Hydroxylapatit-Struktur. Die Zusammensetzung von Knochenmineralien entspricht dabei allerdings nicht der idealen stöchiometrischen Zusammensetzung von kristallinem Hydroxylapatit (Ca₁₀(PO₄)₆(OH)₂), sondern weist in der Regel eine nicht-stöchiometrische Zusammensetzung auf, die durch den Einbau von anderen Anionen wie Carbonat oder Hydrogenphosphat anstelle von Orthophosphat aber auch von anderen Kationen wie Natrium, Kaum oder Magnesium anstelle von Calcium verursacht wird.

Seit einigen Jahren ist man in der Lage, synthetisches Knochenmaterial auf Basis von Hydroxylapatit-ähnlichen Calciumphophatverbindungen herzustellen, das aufgrund seiner qualitativen und strukturellen Ähnlichkeit dem natürlichen Knochen sehr nahe kommt. Damit können die bekannten Nachteile vermieden werden, die durch die Beschaffung von natürlichem autogenen oder heterogenen Knochen entstehen können Synthetische Calciumphosphat-Zemente können vielfältig und unterschiedlich eingesetzt werden, so zum Beispiel bei Knochenfrakturen, Fixierung von Metallprothesen und Knochenschrauben, im Dentalbereich und in der plastischen Chirurgie.

Die Eigenschaften dieser synthetischen Hydroxylapatite bzw. Calciumphosphatzemente (CPC), insbesondere ihre physiologische Akzeptanz, die geforderte Bioresorbierbarkeit und die Fähigkeit durch neu generiertes natürliches Knochengewebe, bzw. Stimulation des Wachstums desselben, ersetzt zu werden, hängen von dem mehr oder weniger stark ausgeprägten Kristallisationsgrad, der Partikelgröße sowie der Porösität ab, die bei der Herstellung erzielt werden können.
Ferner haben diese Materialien den Vorteil, daß sie mechanischen Belastungen praktisch genau so gut widerstehen wie der natürliche Knochen, was ihren Einsatz bei größeren Knochendefekten oder -frakturen nahelegt.

Die Hauptkomponenten dieser Materialien sind beispielsweise Tricalciumphosphat (alpha- und beta-TCP), Dicalciumphosphat (DCP) und Tetracalciumphosphat (TTCP), welche in Anwesenheit von Wasser zu Hydroxylapatit reagieren, dem Endprodukt der Zementbildungs-Reaktion. Da derartig gebildetes Hydroxylapatit in wäßriger Umgebung entstanden ist, ähnelt es den biologischen Apatiten weit mehr als dem Hydroxylapatit, welches bei hohen Temperaturen erzeugt wird. Daher sind derartige Zemente osteotransduktiv und somit sehr gut zur Reparatur und Rekonstruktion von Knochen geeignet. Sie werden schnell in Knochenstrukturen integriert und danach durch die zelluläre Aktivität der Osteoblasten zu neuem Knochengewebe umgewandelt.

Derartige Zemente sind beispielsweise bekannt aus der US 4,518,430, der US 4,612,053, der US 4,678,355, der US 4,880,610, der US 5,053,212, der US 5,152,836, der US 5,605,713, der EP 0416 761, der EP 0 543 765, der EP 0664 133 oder der WO 96/36562. Aus der WO 96/36562 ist ein Hydroxylapatit-Knochenzement (α-BSM) bekannt, welcher aufgrund seiner nahezu amorphen Struktur eine ausgezeichnete Bioresorbierbarkeit und trotz seiner Porösität eine gute mechanische Stabilität aufweist. Dieses sowie viele der in den oben genannten Schriften beschriebenen Materialien lassen sich in Form von aushärtbaren Pasten herstellen, die leicht mittels einer Spritze in den defekten Knochen eingebracht werden können.

Es hat sich aber nun gezeigt, daß derartige Pasten oft teilweise oder ganz desintegrieren bzw. sich inhomogen verhalten, sobald sie mit Körperflüssigkeiten oder anderen wäßrigen Lösungen in Berührung kommen (Jansen et al., 1995, J. Mater. Sci. Mat. Med. 6, 653; Ishikawa et al., 1995, J. Mater. Sci. Mat. Med. 6, 528; Kurashina et al., 1997, Biomaterials 18, 539). Ferner entmischen sich derartige Pasten leicht während der Extrudierens aus Spritzen, wobei der flüssigere Teil aus der Spritze gepreßt wird, während der festere Teil in der Spritze verbleibt und auch durch höhere Preßdrucke nicht aus ihr entfernt werden kann. Durch das Entmischen kann somit ein Material erhalten werden, das nicht mehr für den beabsichtigten Zweck geeignet ist.

Cherng et al. (J. Biomed. Mat. Res. 35, 1997, S. 273) und Chow et al. (Innov. Techn. Biol. Med. 18, 1997, S. 11) berichten über den Einsatz von Hydroxypropylmethyl- und Carboxymethylcellulose sowie Chitosanderivaten bei Calciumphosphat-Zementen auf Basis von Tetracalciumphosphat (TTCP). Die Zusätze führen zu verbesserten Verarbeitungseigenschaften hinsichtlich der Kohäsion der verwendeten Zementpasten, bewirken jedoch häufig verschlechterte Aushärtungskinetiken und reduzierte mechanische Festigkeit der ausgehärteten Zemente.

Es bestand somit die Aufgabe, Zementpasten, die zu aushärtbaren, biokompatiblen synthetischen Knochenzementen führen, zur Verfügung zu stellen, die nicht nur deutlich verbesserte Kohäsionseigenschaften aufweisen sondern auch ausreichende bzw. günstige Injizierbarkeit, mechanische Härte und Aushärtungszeiten besitzen.

Die Aufgabe wurde durch die Pasten der vorliegenden Erfindung gelöst.

Gegenstand der Erfindung ist somit eine verarbeitungsfähige, aushärtbare Paste, geeignet für die Herstellung biokompatibler Knochenzemente, die durch Vermischen eines Zementpulvers, welches Tricalciumphosphat (TCP) und mindestens eine weitere calciumphosphathaltige Verbindung enthält, mit einer wäßrigen Lösung eines Kohäsionspromotors und eines Aushärtungsbeschleunigers erhalten werden kann.

Die Kohäsionspromotoren werden erfindungsgemäß in einer Konzentration von 0,1 bis 10%, vorzugsweise von 1 bis 6% bezogen auf die wäßrige Lösung eingesetzt. Die entsprechenden Verbindungen werden dabei in Wasser gelöst. Gegebenenfalls können kleine Anteile an organischen Lösungsmitteln zugegen sein, falls der Kohäsionspromotor in reinem Wasser schlecht löslich ist.

Als Kohäsionspromotoren kommen prinzipiell alle für diese Aufgabe bekannten Verbindungen, insbesondere oligomere und/oder polymere Verbindungen in Frage, vorzugsweise werden Verbindungen ausgesucht aus der Gruppe der Cellulosen, beispielsweise Hydroxyethyl-, Hydroxymethyl-, Hydroxypropylmethyl- oder auch Carboxymethylcellulose, vorzugsweise Hydroxyethylstärke und lösliche Stärke, Cyclodextrine, wie α-, β- oder γ-Cyclodextrin, Alginate, wie Natriumalginat, Dextransulfate, wie Natriumdextransulfat, Polyvinylpyrrolidon und Hyaluronsäure. Erfindungsgemäß können auch mehrere Kohäsionspromotoren eingesetzt werden.

Die erfindungsgemäßen Pasten enthalten weiterhin Aushärtungsbeschleuniger, beispielsweise Na₂HPO₄, in Konzentrationen zwischen 0,5 und 5%. Die Konzentration richtet sich nach der Wahl des Kohäsionspromoters aber vor allem nach der Zusammensetzung des Calciumphosphat-Zements in Verbindung mit der gewünschten medizinischen Applikation.

Für die Kohäsions- und Injizierbarkeitseigenschaften der erfindungsgemäßen Pasten ist ferner das Verhältnis zwischen Flüssigkeit und Zementpulver, die miteinander vermischt werden, von Bedeutung. Vorzugsweise ist das Verhältnis zwischen wäßriger Lösung und Zementpulver zwischen 0,30 und 0,45 ml/g, insbesondere zwischen 0,35 und 0,40 ml/g.

Überraschend hat sich gezeigt, daß die bekannten negativen Eigenschaften von ähnlichen bekannten Materialien des Standes der Technik bei Einsatz andere, wenn auch ähnlicher kohäsionsfördernder Verbindungen, insbesondere im Hinblick auf Festigkeit und verlängerte Aushärtungszeiten, vermieden oder abgeschwächt werden können, wenn die Zementmischung Tricalciumphosphat (TCP), insbesondere α-TCP, enthält. Vorzugsweise ist mindestens eine weitere calciumphophathaltige Verbindung, vorzugsweise präzipitiertes Hydroxylapatit (PHA) zugegen. Zementmischungen, die also α-TCP und PHA enthalten sind bevorzugt. Ferner sind Mischungen die α-TCP, PHA und Dicalciumphosphat (DCP) enthalten, bevorzugt, ebenso Mischungen, die jeweils zusätzlich Calciumcarbonat enthalten. Durch Zusatz der genannten Aushärtungsbeschleuniger in unterschiedlichen Konzentrationen können bei den erfindungsgemäßen Pasten in individueller Weise je nach gewünschter klinischer Anwendung schneller oder langsamer aushärtbare Zemente mit ausreichend großer mechanischer Festigkeit nach erfolgter Aushärtung gewonnen werden.

Gegenstand der Erfindung ist somit eine entsprechende Paste, worin das Calciumphospat-Zementpulver folgende Bestandteile aufweist:
(i) α-Tricalciumphosphat (α-TCP), präzipitiertes Hydroxylapatit (PHA) oder
(ii) α-TCP, PHA, Dicalciumphosphat (DCP) oder
(iii) α-TCP, PHA, DCP, CaCO₃ oder
(iv) α-TCP, PHA, CaCO₃.

Insbesondere ist Gegenstand der Erfindung eine entsprechende Paste, worin das Calciumphospat-Zementpulver folgende Bestandteile aufweist:
(i) α-TCP (98%), PHA (2%) oder
(ii) α-TCP (64%), PHA(9%), DCP (27%) oder
(iii) α-TCP (58%), PHA (8,5%), DCP (25%), CaCO₃ (8,5%) oder
(iv) α-TCP (90%), PHA (5%), CaCO₃ (5%).

Die erfindungsgemäßen Pasten werden vorzugsweise in der Weise hergestellt, daß die Kohäsionspromotoren und Aushärtungsbeschleuniger in den gewünschten genannten Konzentrationen in Wasser oder wasserhaltigen Gemischen gelöst wird. Diese Zementflüssigkeit wird dann mit dem Zementpulvergemisch vermischt, bzw. zu der besagten Paste angerührt.

Gegenstand der Erfindung ist somit schließlich ein Verfahren zur Herstellung einer calciumphosphathaltigen Zementpaste mit verbesserten Kohäsionseigenschaften, welches dadurch gekennzeichnet ist, daß man ein Zementpulver, welches Tricalciumphosphat (TCP) und mindestens eine weitere calciumphosphathaltige Verbindung enthält, mit einer wäßrigen Lösung eines Kohäsionspromotors in einer Konzentration von 0,1 bis 5%, bezogen auf die flüssige Phase, vermischt, wobei je nach gewünschter medizinischer Anwendung die Aushärtungskinetik durch Einsatz eines Aushärtungsbeschleunigers in einer Konzentration von 0,2 bis 5% bezogen auf die flüssige Phase individuell eingestellt werden kann.

Unter dem Begriff "Kohäsionszeit" (CT) wird erfindungsgemäß die Zeitspanne zwischen Mischen des Pulvers und der Flüssigkeit und dem Moment verstanden, in dem die Paste nicht mehr desintegriert, wenn sie 24 h lang in Ringer'scher Lösung eingetaucht ist (Fernandez et al., 1996, J. Mater. Sci. Letters 15, 1004).

Unter dem Begriff "Injizierbarkeit" wird erfindungsgemäß der prozentuale Gewichtsanteil der Menge an Calciumphosphat-Paste zwischen 2 und 4 g verstanden, welche aus einer 20 ml Spritze mit einer Kraft von maximal 100 N innerhalb von 2 min ab Beginn des Vermischens extrudiert wird.

Bei der Aushärtungszeit werden zwei Begriffe unterschieden und zwar die Zeit, bei der die Aushärtung begonnen hat ("Initial Setting Time", IT) und der Zeitpunkt, bei die Aushärtung abgeschlossen ist ("Final Setting Time", FT). Die IT wird erfindungsgemäß mit Hilfe der dicken und leichten Gilmore-Nadel, die FT mit der dünnen und schweren Gilmore-Nadel gemessen (Driessens et al, 1993, J. Mater. Sci. Mat. Med. 4, 503).

Die mechanische Festigkeit oder kompressive Härte wird erfindungsgemäß nach drei Tage langem Eintauchen der ausgehärteten Paste in Ringer'scher Lösung bei 37° C gemessen (Bermudez et al., 1993, J. Mater. Sci. Mat. Med. 4, 389).

Gemäß der Erfindung werden die vorzugsweise oligomeren / polymeren Kohäsionspromotoren sowie der oder die Aushärtungsbeschleuniger in der Zementflüssigkeit gelöst oder suspendiert und diese Lösung mit dem Pulvergemisch auf Basis von TCP zu einer verarbeitungsfähigen Paste vermischt. Eine verarbeitungsfähige Paste kann nur erhalten werden, wenn ein bestimmtes Verhältnis zwischen Flüssigkeit und Pulver eingehalten wird. Dieses Verhältnis (ml/g) liegt erfindungsgemäß zwischen 0,25 und 0,50, vorzugsweise zwischen 0,3 und 0,45, besonders bevorzugt zwischen 0,35 und 0,40. Nach dem Vermischen zu einer verarbeitungsfähigen Paste kann diese entweder direkt in den Implantationsort eingebracht oder in eine Spritze eingefüllt werden, aus der sie in den Implantationsort injiziert wird, ehe die "initial setting time" erreicht wird, also ehe die Paste auszuhärten beginnt.

Im folgenden wird die Erfindung anhand von Beispielen näher beschrieben, ohne dabei beschränkend zu sein. Die Zusammensetzung der verwendeten Calciumphosphat-Zemente (CPC) ist aus Tabelle 1 zu entnehmen.

**Tab 1**

| CPC | Prozentualer Anteil (Gew. %) | | | |
|---|---|---|---|---|
| | α-TCP | PHA | DCP | CaCO₃ |
| Biozement H | 98 | 2 | | |
| Biozement F | 64 | 9 | 27 | |
| Biozement B | 90 | 5 | | 5 |
| Biozement D | 58 | 8,5 | 25 | 8,5 |

In allen Beispielen, in denen Kohäsionspromotoren zugefügt sind, wird Na₂HPO₄ (1%) als Aushärtungsbeschleuniger verwendet. In diesen Beispielen wird der Einfluß der verschiedenen Kohäsionspromotoren auf die angegebenen Biozemente untersucht. Die gemessenen Parameter sind dabei jeweils:
- L/P =: Verhältnis Flüssigkeit / Pulver in ml / g;
- CT =: Kohäsionszeit in min (wie oben definiert);
- IT =: "initial setting time" in min (wie oben definiert);
- FT =: "final setting time" in min (wie oben definiert);
- CS =: "compressive strength" (Druckfestigkeit) in MPa (wie oben definiert);
- IJ =: Injektizierbarkeit in % (wie oben definiert).

Die mechanische Festigkeit der ausgehärteten Zemente ist weitgehend unabhängig von der Konzentration des Aushärtungsbeschleunigers (zumindest bis 4%). Eine Ausnahme hiervon liegt beim Biozement H vor. So verringert sich der CS-Wert von 55 MPa bei 0% Na₂HPO₄ kontinuierlich auf 27 MPa bei 4% Na₂HPO₄.

### Beispiel 1:

Einfluß von Hydroxylethylstärke (HES) (1,5 Gew. %, bezogen auf die Gesamtmenge an Zementpulver) auf verschiedene Biozemente.

| **Biozement** | **L/P**(ml/g) | **HES** | **CT**(min) | **IT**(min) | **FT**(min) | **CS**(MPa) | **IJ**(%) |
|---|---|---|---|---|---|---|---|
| **H** | 0,35 | - | 8 | 12 | 30 | 48 | 53 |
| | | + | 1,3 | 14,5 | >45 | 37 | 60 |
| | 0,40 | | 11 | 20 | 58 | 35 | 75 |
| | | | 1,3 | 22 | >45 | 34 | 78 |
| **F** | 0,35 | - | 8 | 10 | 20 | 33 | 89 |
| | | + | 4 | 11 | 26 | 27 | 89 |
| | 0,40 | - | 10 | 12,5 | 29 | 22 | 93 |
| | | + | 1,5 | 16 | >45 | 23 | 94 |
| **B** | 0,35 | - | 9 | 16 | 34 | 43 | 76 |
| | | + | 1,75 | 14,5 | 32 | 56 | 72 |
| | 0,40 | - | 13,5 | 21 | 42 | 43 | 90 |
| | | + | 1,5 | 19 | >45 | 43 | 90 |
| **D** | 0,35 | - | 10 | 16 | 24 | 45 | 91 |
| | | + | 1,25 | 12 | 27,5 | 42 | 91 |
| | 0,40 | - | 13 | 18 | 34 | 38 | 94 |
| | | + | 1,5 | 18,5 | 50 | 33 | 91 |

### Beispiel 2:

Einfluß von Natriumdextransulfat (SDS) (4 Gew. %, bezogen auf die Gesamtmenge an Zementpulver) auf verschiedene Biozemente.

| **Biozement** | **L/P**(ml/g) | **SDS** | **CT**(min) | **IT**(min) | **FT**(min) | **CS**(MPa) | **IJ**(%) |
|---|---|---|---|---|---|---|---|
| **H** | 0,35 | - | 8 | 12 | 30 | 39 | 53 |
| | | + | 1,25 | 8,5 | 25 | 30 | 36 |
| | 0,40 | | 11 | 20 | 58 | 30 | 75 |
| | | | 1,25 | 13 | 35 | 27 | 88 |
| **F** | 0,35 | - | 8 | 10 | 20 | 29 | 89 |
| | | + | 3 | 8 | 31 | 37 | 91 |
| | 0,40 | - | 10 | 12,5 | 29 | 22 | 93 |
| | | + | 2 | 10 | 38 | 28 | 90 |
| **B** | 0,35 | - | 9 | 16 | 34 | 24 | 76 |
| | | + | 1,5 | 8 | 30 | 33 | 82 |
| | 0,40 | - | 13,5 | 21 | 42 | 21 | 90 |
| | | + | 1,25 | 10 | 38 | 28 | 92 |
| **D** | 0,35 | - | 10 | 16 | 24 | 40 | 91 |
| | | + | 4 | 5,5 | 14 | 36 | 82 |
| | 0,40 | - | 13 | 18 | 34 | 31 | 94 |
| | | + | 4 | 7 | 23 | 32 | 91 |

### Beispiel 3:

Einfluß von ß-Cyclodextrin (ß-CD) (1 Gew, %, bezogen auf die Gesamtmenge an Zementpulver) auf verschiedene Biozemente.

| **Biozement** | **L/P**(ml/g) | **ß-CD** | **CT**(min) | **IT**(min) | **FT**(min) | **CS**(MPa) | **IJ**(%) |
|---|---|---|---|---|---|---|---|
| **H** | 0,35 | - | 8 | 12 | 30 | 39 | 53 |
| | | + | 1,25 | 10,5 | 26 | 37 | 57 |
| | 0,40 | | 11 | 20 | 58 | 30 | 75 |
| | | | 1,25 | 15 | 45 | 32 | 83 |
| **F** | 0,35 | - | 8 | 10 | 20 | 29 | 89 |
| | | + | 1,5 | 10,5 | 20,5 | 38 | 81 |
| | 0,40 | - | 10 | 12,5 | 29 | 22 | 93 |
| | | + | 2 | 13 | 32 | 27 | 88 |
| **B** | 0,35 | - | 9 | 16 | 34 | 24 | 76 |
| | | + | 4,5 | 9 | 19 | 33 | 79 |
| | 0,40 | - | 13,5 | 21 | 42 | 21 | 90 |
| | | + | 1,25 | 12,5 | 37 | 26 | 89 |
| **D** | 0,35 | - | 10 | 16 | 24 | 40 | 91 |
| | | + | 1,25 | 8 | 15 | 39 | 89 |
| | 0,40 | - | 13 | 18 | 34 | 31 | 94 |
| | | + | 2 | 11 | 22,5 | 34 | 93 |

### Beispiel 4:

Einfluß von Natriumalginat (SALG) (1 Gew. %, bezogen auf die Gesamtmenge an Zementpulver) auf verschiedene Biozemente.

| **Biozement** | **L/P**(ml/g) | **SALG** | **CT**(min) | **IT**(min) | **FT**(min) | **CS**(MPa) | **IJ**(%) |
|---|---|---|---|---|---|---|---|
| **H** | 0,35 | - | 8 | 12 | 30 | 39 | 53 |
| | | + | 1,5 | 14,5 | 44 | 27 | 36 |
| | 0,40 | | 11 | 20 | 58 | 30 | 75 |
| | | | 1,5 | 20,5 | >40 | 26 | 87 |
| **F** | 0,35 | - | 8 | 10 | 20 | 29 | 89 |
| | | + | 1,5 | 10 | 23 | 17 | 90 |
| | 0,40 | - | 10 | 12,5 | 29 | 22 | 93 |
| | | + | 1,5 | 15,5 | 38 | 19 | 93 |
| **B** | 0,35 | - | 9 | 16 | 34 | 24 | 76 |
| | | + | 1,5 | 9,5 | 23 | 24 | 83 |
| | 0,40 | - | 13,5 | 21 | 42 | 21 | 90 |
| | | + | 2 | 10,5 | 31,5 | 24 | 94 |
| **D** | 0,35 | - | 10 | 16 | 24 | 40 | 91 |
| | | + | 1,5 | 12,5 | 28 | 34 | 83 |
| | 0,40 | - | 13 | 18 | 34 | 31 | 94 |
| | | + | 1,5 | 17 | 42 | 32 | 89 |

### Beispiel 5:

Einfluß von löslicher Stärke (SST) (2 Gew. %, bezogen auf die Gesamtmenge an Zementpulver) auf verschiedene Biozemente.

| **Biozement** | **L/P**(ml/g) | **SST** | **CT**(min) | **IT**(min) | **FT**(min) | **CS**(MPa) | **IJ**(%) |
|---|---|---|---|---|---|---|---|
| **H** | 0,35 | - | 8 | 12 | 30 | 39 | 53 |
| | | + | 5 | 15 | >40 | 35 | 51 |
| | 0,40 | | 11 | 20 | 58 | 30 | 75 |
| | | | 2 | 20 | >40 | 26 | 77 |
| **F** | 0,35 | - | 8 | 10 | 20 | 29 | 89 |
| | | + | 5 | 8,5 | 26 | 38 | 83 |
| | 0,40 | - | 10 | 12,5 | 29 | 22 | 93 |
| | | + | 1,5 | 14 | 37,5 | 30 | 89 |
| **B** | 0,35 | - | 9 | 16 | 34 | 32 | 76 |
| | | + | 5 | 9,5 | 21 | 44 | 88 |
| | 0,40 | - | 13,5 | 21 | 42 | 43 | 90 |
| | | + | 1,5 | 12 | 32 | 36 | 90 |
| **D** | 0,35 | - | 10 | 16 | 24 | 40 | 91 |
| | | + | 1 | 11 | 27 | 47 | 88 |
| | 0,40 | - | 13 | 18 | 34 | 31 | 94 |
| | | + | 1 | 16 | 38 | 38 | 91 |

### Beispiel 6:

Einfluß von Polyvinylpyrrolidon (PVP) (6 Gew. %, bezogen auf die Gesamtmenge an Zementpulver) auf verschiedene Biozemente.

| **Biozement** | **L/P**(ml/g) | **PVP** | **CT**(min) | **IT**(min) | **FT**(min) | **CS**(MPa) | **IJ**(%) |
|---|---|---|---|---|---|---|---|
| **H** | 0,35 | - | 8 | 12 | 30 | 50 | 53 |
| | | + | 1,5 | 12 | >40 | 44 | 49 |
| | 0,40 | | 11 | 20 | 58 | 39 | 75 |
| | | | 1,25 | 13 | >40 | 42 | 86 |
| **F** | 0,35 | - | 8 | 10 | 20 | 29 | 89 |
| | | + | 5 | 8 | 26 | 30 | 86 |
| | 0,40 | - | 10 | 12,5 | 29 | 22 | 93 |
| | | + | 1,25 | 8,5 | 42 | 24 | 92 |
| **B** | 0,35 | - | 9 | 16 | 34 | 32 | 76 |
| | | + | 5 | 9 | 32 | 32 | 72 |
| | 0,40 | - | 13,5 | 21 | 42 | 43 | 90 |
| | | + | 5 | 10,5 | 43 | 34 | 92 |
| **D** | 0,35 | - | 10 | 16 | 24 | 47 | 91 |
| | | + | 5 | 9 | 29 | 35 | 89 |
| | 0,40 | - | 13 | 18 | 34 | 33 | 94 |
| | | + | 1,5 | 11 | 40 | 35 | 92 |

### Beispiel 7:

Einfluß von Kaliumhyaluronat (HAPS) (0,2 Gew. %, bezogen auf die Gesamtmenge an Zementpulver) auf verschiedene Biozemente.

| **Biozement** | **L/P**(ml/g) | **HAPS** | **CT**(min) | **IT**(min) | **FT**(min) | **CS**(MPa) | **IJ**(%) |
|---|---|---|---|---|---|---|---|
| **H** | 0,35 | - | 8 | 12 | 30 | 39 | 53 |
| | | + | 1,75 | 12,5 | >40 | 35 | 56 |
| | 0,40 | | 11 | 20 | 58 | 30 | 75 |
| | | | 1,5 | 17 | >40 | 33 | 89 |
| **F** | 0,35 | - | 8 | 10 | 20 | 29 | 89 |
| | | + | 1,5 | 8,5 | 24 | 37 | 82 |
| | 0,40 | - | 10 | 12,5 | 29 | 22 | 93 |
| | | + | 1,75 | 10 | 33 | 27 | 90 |
| **B** | 0,35 | - | 9 | 16 | 34 | 24 | 76 |
| | | + | 5 | 9 | 23,5 | 37 | 85 |
| | 0,40 | - | 13,5 | 21 | 42 | 21 | 90 |
| | | + | 1,5 | 11 | 31 | 32 | 92 |
| **D** | 0,35 | - | 10 | 16 | 24 | 40 | 91 |
| | | + | 4 | 8 | 22 | 39 | 91 |
| | 0,40 | - | 13 | 18 | 34 | 31 | 94 |
| | | + | 3 | 9,5 | 34 | 36 | 94 |

### Beispiel 8:

Einfluß von α-Cyclodextrin (α-CD) (4 Gew. %, bezogen auf die Gesamtmenge an Zementpulver) auf verschiedene Biozemente.

| **Biozement** | **L/P(**ml/g) | **α-CD** | **CT(**min) | **IT**(min) | **FT**(min) | **CS**(MPa) | **IJ**(%) |
|---|---|---|---|---|---|---|---|
| **H** | 0,35 | - | 8 | 12 | 30 | 48 | 53 |
| | | + | 1,75 | 13,5 | >40 | 39 | 50 |
| | 0,40 | | 11 | 20 | 58 | 35 | 75 |
| | | | 1,75 | 14,5 | >40 | 37 | 79 |
| **F** | 0,35 | - | 8 | 10 | 20 | 29 | 89 |
| | | + | 4 | 8 | 28 | 31 | 83 |
| | 0,40 | - | 10 | 12,5 | 29 | 22 | 93 |
| | | + | 2 | 13 | 34,5 | 23 | 90 |
| **B** | 0,35 | - | 9 | 16 | 34 | 38 | 76 |
| | | + | 2,5 | 8 | 23 | 50 | 82 |
| | 0,40 | - | 13,5 | 21 | 42 | 43 | 90 |
| | | + | 1,5 | 12 | 39 | 38 | 89 |
| **D** | 0,35 | - | 10 | 16 | 24 | 45 | 91 |
| | | + | 2 | 8 | 24 | 46 | 89 |
| | 0,40 | - | 13 | 18 | 34 | 38 | 94 |
| | | + | 1,5 | 15 | 41,5 | 33 | 91 |

### Beispiel 9:

Einfluß von γ-Cyclodextrin (γ-CD) (2,5 Gew. %, bezogen auf die Gesamtmenge an Zementpulver) auf verschiedene Biozemente.

| **Biozement** | **L/P**(ml/g) | **γ-CD** | **CT(**min) | **IT**(min) | **FT**(min) | **CS**(MPa) | **IJ**(%) |
|---|---|---|---|---|---|---|---|
| **H** | 0,35 | - | 8 | 12 | 30 | 39 | 53 |
| | | + | 1,75 | 11 | >40 | 30 | 53 |
| | 0,40 | | 11 | 20 | 58 | 30 | 75 |
| | | | 1,75 | 14 | >40 | 34 | 80 |
| **F** | 0,35 | - | 8 | 10 | 20 | 29 | 89 |
| | | + | 3 | 8,5 | 24 | 27 | 85 |
| | 0,40 | - | 10 | 12,5 | 29 | 22 | 93 |
| | | + | 1,5 | 10 | 33 | 21 | 92 |
| **B** | 0,35 | - | 9 | 16 | 34 | 24 | 76 |
| | | + | 3 | 9 | 20,5 | 35 | 85 |
| | 0,40 | - | 13.5 | 21 | 42 | 21 | 90 |
| | | + | 1,75 | 12 | 43 | 28 | 95 |
| **D** | 0,35 | - | 10 | 16 | 24 | 40 | 91 |
| | | + | 4 | 8,5 | 20 | 39 | 94 |
| | 0,40 | - | 13 | 18 | 34 | 31 | 94 |
| | | + | 1,75 | 9,5 | 28 | 30 | 91 |

## Patentansprüche

1. Verarbeitungsfähige, aushärtbare Paste, geeignet für die Herstellung biokompatibler Knochenzemente, erhältlich durch Vermischen eines Zementpulvers, welches Tricalciumphosphat (TCP) und mindestens eine weitere calciumphosphathaltige Verbindung enthält, mit einer wäßrigen Lösung eines Kohäsionspromotors und eines Aushärtungsbeschleunigers.

2. Paste nach Anspruch 1, worin das verwendete Zementpulver TCP und präzipiziertes Hydroxylapatit (PHA) enthält.

3. Paste nach Anspruch 1 oder 2, dadurch gekennzeichnet, daß der Kohäsionspromotor in einer Konzentration von 0,1 bis 10 % bezogen auf die flüssige Phase vorliegt.

4. Paste nach einem der Ansprüche 1 bis 3, dadurch gekennzeichnet, daß als Kohäsionspromotor eine Verbindung ausgewählt wird aus der Gruppe Hydroxyethylstärke, lösliche Stärke, Cyclodextrine, Alginate, Dextransulfate, Polyvinylpyrrolidon und Hyaluronsäure.

5. Paste nach einem der Ansprüche 1 bis 4, dadurch gekennzeichnet, daß als Aushärtungsbeschleuniger Dinatriumhydrogenphosphat in einer Konzentration von 0,5 bis 5% bezogen auf die flüssige Phase vorliegt.

6. Paste nach einem der Ansprüche 1 bis 5, dadurch gekennzeichnet, daß das Verhältnis zwischen wäßriger Lösung und Zementpulver 0,30 bis 0,45 ml/g beträgt.

7. Paste nach einem der Ansprüche 1 bis 6, dadurch gekennzeichnet, daß das Calciumphospat-Zementpulver folgende Bestandteile aufweist:
(v) α-Tricalciumphosphat (α-TCP), präzipitiertes Hydroxylapatit (PHA) oder
(vi) α-TCP, PHA, Dicalciumphosphat (DCP) oder
(vii) α-TCP, PHA, DCP, CaCO₃ oder
(viii) α-TCP, PHA, CaCO₃.

8. Paste nach Anspruch 7, dadurch gekennzeichnet, daß das Calciumphospat-Zementpulver folgende Zusammensetzung aufweist:
(v) α-TCP (98%), PHA (2%) oder
(vi) α-TCP (64%), PHA(9%), DCP (27%) oder
(vii) α-TCP (58%), PHA (8,5%), DCP (25%), CaCO₃ (8,5%) oder
(viii) α-TCP (90%), PHA (5%), CaCO₃ (5%).

9. Verfahren zur Herstellung einer calciumphosphathaltigen Zementpaste mit verbesserten Kohäsionseigenschaften, dadurch gekennzeichnet, daß man ein Zementpulver, welches Tricalciumphosphat (TCP) und mindestens eine weitere calciumphosphathaltige Verbindung enthält, mit einer wäßrigen Lösung eines Kohäsionspromotors in einer Konzentration von 0,1 bis 5%, bezogen auf die flüssige Phase, vermischt, wobei je nach gewünschter medizinischer Anwendung die Aushärtungskinetik durch Einsatz eines Aushärtungsbeschleunigers in einer Konzentration von 0,2 bis 5% bezogen auf die flüssige Phase individuell eingestellt werden kann.

10. Verfahren nach Anspruch 9, dadurch gekennzeichnet, daß das Verhältnis zwischen wäßriger Lösung und Zementpulver 0,30 bis 0,45 ml / g beträgt und als Kohäsionspromotor eine Verbindung aus der Gruppe Hydroxyethylstärke, lösliche Stärke, Cyclodextrine, Alginate, Dextransulfate, Polyvinylpyrrolidon und Hyaluronsäure und als Aushärtungsbeschleuniger Dinatriumhydrogenphospat eingesetzt wird.
